**Europäisches Patentamt**

**European Patent Office**

**Office européen des brevets**

(19)

(11) Veröffentlichungsnummer: **0 231 838**

**A2**

## (12) EUROPÄISCHE PATENTANMELDUNG

(21) Anmeldenummer: **87100974.2**

(51) Int. Cl.⁴: $A61C\ 8/00$

(22) Anmeldetag: **23.01.87**

---

Die Bezeichnung der Erfindung wurde geändert (Richtlinien für die Prüfung im EPA, A-III, 7.3).

(30) Priorität: **30.01.86 DE 3602721**

(43) Veröffentlichungstag der Anmeldung:
**12.08.87 Patentblatt 87/33**

(84) Benannte Vertragsstaaten:
**AT BE CH DE ES FR GB GR IT LI LU NL SE**

(71) Anmelder: **Feldmühle Aktiengesellschaft
Fritz-Vomfelde-Platz 4
D-4000 Düsseldorf 11(DE)**

(72) Erfinder: **von Chamier, Wilfried
Botnanger Steige 18
D-7000 Stuttgart(DE)**
Erfinder: **Tetsch, Peter, Prof. Dr. Dr.
Hohe Geist 72
D-4400 Münster(DE)**

---

(54) **Zahnimplantat.**

(57) Ein endodontisches Implantat (1, 11, 17) aus einem biologisch verträglichen Material besitzt zur Herstellung eines kontrastreichen Röntgenbildes mindestens ein Markierungsteil (4, 4') aus einem Material mit hohem Röntgenkontrast.

**Fig. 1**

EP 0 231 838 A2

Die vorliegende Erfindung betrifft ein Zahnimplantat entsprechend dem Gattungsbegriff des Hauptanspruches.

In dem DE-Buch "Chirurgische Zahnheilkunde", Thieme-Verlag (1980), s. Seite 212, wird die Wurzelspitzenresektion bei erhaltungswürdigen Zähnen beschrieben. Die dort beschriebene Operationstechnik sieht eine Wurzelfüllung mit Silberamalgam vor. In neuerer Zeit sind auch vorgefertigte Wurzelkanalstifte bekannt geworden, wie sie z.B. in der DE-A-26 09 243 beschrieben werden. Der dort beschriebene Wurzelkanalstift zum Verschließen eines Wurzelkanals besteht aus Titan.

Ein endodontisches Implantat ist auch aus der DE-A-29 36 690 bekannt. Der Schaft des dort beschriebenen Implantates ist zur Implantation durch den Wurzelkanal eines Zahnstumpfes und durch die Spitze dieses Kanals in den benachbarten Knochen passend ausgebildet, während ein mit dem Schaft integral verbundenes Kronenteil der Befestigung einer Zahnkrone dient.

Diese Schrift gibt keinen Hinweis auf die Art der verwendeten Materialien.

Der Nachteil der bekannten endodontischen Metallimplantate liegt in ihrer noch nicht in jedem Fall ausreichenden Gewebeverträglichkeit. Dieser Nachteil besteht nicht bei den aus dem Stand der Technik bekannten Kieferimplantaten aus Keramik, insbesondere aus Aluminiumoxidkeramik. Diese Implantate weisen eine hervorragende biologische Verträglichkeit auf und man hat auch bereits versucht, endodontische Implantate aus diesen Werkstoffen herzustellen, wie sich aus der JP-Firmenshrift: "Bioceram" der Firma Kyocera ergibt. Dabei besteht jedoch der Nachteil, daß diese Implantate in der Regel nur einen sehr schwachen Röntgenkontrast ergeben, wobei sich dieser Nachteil insbesondere dann auswirkt, wenn, wie bei endodontischen Implantaten, das Implantat noch von der Dentinmasse des Zahns umgeben ist. Die sachgerechte Beurteilung eines Röntgenbildes bereitet daher Schwierigkeiten und kann insbesondere dann zu Problemen führen, wenn ein ein Implantat tragender Patient von einem Arzt behandelt wird, der das Implantat nicht gesetzt hat.

Aus der DE-A-24 47 787 sind auch bereits oxidkeramische Implantate bekannt, die aufgrund ihrer materialmäßigen Zusammensetzung, nämlich durch den Zusatz von $ZrO_2$, $TiO_2$ und $Y_2O_3$, über einen im Vergleich zu $Al_2O_3$-Keramik erhöhten Röntgenkontrast verfügen sollen. Dieser Vorschlag hat sich jedoch nicht durchsetzen können, weil sich in der Zwischenzeit lediglich aus $Al_2O_3$ bestehende Zusammensetzungen auf breiter Basis durchgesetzt haben. Neben der wahrscheinlich nicht ausreichenden biologischen Verträglichkeit der in der DE-A-24 47 787 genannten Zusatzstoffe dürften vor allem die nicht ausreichende Festigkeit bei Zusatz von $TiO_2$, die einsetzende Gelbfärbung bei Zusatz von $ZrO_2$ und die hohen Kosten von $Y_2O_3$ die Gründe dafür sein, daß dieser bekannte Vorschlag keine weitere Verbreitung gefunden hat.

Die vorliegende Erfindung hat sich die Aufgabe gesetzt, diese Problematik zu überwinden und ein Zahnimplantat zu schaffen, das sowohl über eine ausgezeichnete biologische Verträglichkeit verfügt als auch ganz allgemein dann eine sichere röntgenologische Überprüfung des Implantatsitzes gestattet, wenn das Implantat aufgrund seiner geringen Größe und wenn es vom Knochen umschlossen ist, röntgenologisch nur mit größten Schwierigkeiten zu identifizieren ist. Insbesondere will die Erfindung ein endodontisches Implantat schaffen, dessen Sitz, obwohl das Implantat von der Dentinmasse des Zahnes umgeben ist, röntgenologisch sicher überprüft werden kann.

Zur Lösung dieser Aufgabe sieht die vorliegende Erfindung bei einem Implantat gemäß dem Oberbegriff von Anspruch 1 dessen kennzeichnende Merkmale vor.

Unter dem in Ansprüchen und Beschreibung verwendeten Begriff "Zahnimplantat" sollen sowohl endodontische Implantate, wie z.B. Wurzelkanalstifte, aber auch enossale, d.h. in den Kieferknochen einsetzbare Implantate verstanden werden.

Da der Körper des erfindungsgemäßen Zahnimplantates aus einem biologisch verträglichen Material besteht und nur das Markierungsteil aus einem anderen Werkstoff, nämlich einem Material mit einem hohen Röntgenkontrast, ist sowohl eine hohe biologische Verträglichkeit gegeben als auch die Möglichkeit, die Lage des Implantates nach seiner Implantation auf röntgenologischem Wege sicher zu bestimmen.

Bevorzugt wird als biologisch verträgliches Material Keramik, insbesondere Aluminiumoxidkeramik verwendet. Erfindungsgemäß wird dabei entweder im Inneren des Zahnimplantates ein metallisches Markierungsteil angeordnet, d.h.: das Markierungsteil ist in das Zahnimplantat eingelagert, bevorzugt wird jedoch, daß das Markierungsteil auf der Oberfläche des Zahnimplantates angeordnet ist. Zur Sicherstellung eines hohen Röntgenkontrastes ist dabei die Herstellung des Markierungsteils aus Titan, Gold, Platin oder Edelstahl bevorzugt. Gemäß einer in konstruktiver Hinsicht besonders bevorzugten Ausführungsform besteht das Markierungsteil aus einem Sprengring, der in einer Rille an der Schaftoberfläche des Implantates eingeklemmt ist.

Eine weitere Möglichkeit besteht darin, die Schaftoberfläche des Zahnimplantates in einem bestimmten Bereich zu beschichten und dadurch ein Markierungsteil auf der Oberfläche des Zahnimplantates zu erzeugen.

Die Beschichtung kann nach den bekannten Verfahren der Metallisierung, CVD und PVD-Beschichtung, Flamm-und Plasmabeschichtung erfolgen.

Bei einem als Wurzelkanalstift ausgebildeten Zahnimplantat ist erfindungsgemäß vorgesehen, das Markierungsteil auf dem zur endodontischen Einlagerung vorgesehenen Bereich des Implantates auszubilden. Wird ein solches Implantat in einen Zahnstumpf eingesetzt, ist eine sichere Identifizierung des Zahnimplantates mittels Röntgen möglich, da das Markierungsteil aus z.B. Titan auch dann noch festgestellt wird, wenn die Dentinmasse des Zahnes den Körper des z.B. aus hochreinem Aluminiumoxid bestehenden Zahnimplantates vollständig umhüllt.

Erfindungsgemäß kann der Wurzelkanalstift in Form einer konischen oder zylindrischen Schraube ausgebildet sein, ist aber bevorzugt als glatter und konischer Stift ausgebildet.

Wenngleich die Ausbildung des erfindungsgemäßen Zahnimplantates als endodontischer Wurzelkanalstift besonders bevorzugt ist, ist die Anwendung des Implantates nicht auf dieses Gebiet beschränkt. Gemäß weiteren geeigneten Ausführungsformen kann das Implantat als enossales Kieferimplantat ausgebildet sein. Die Form der jeweiligen Implantate ist dabei nicht kritisch und kann sich mit Einschränkung an den bekannten Implantatformen orientieren.

Anhand der nachfolgenden Figuren wird die Erfindung näher erläutert.

Es zeigen:

Figur 1 eine Perspektivdarstellung und eine Ausschnittvergrößerung des erfindungsgemäßen Zahnimplantates mit noch nicht eingesetztem Ring

Figur 2 eine Perspektivdarstellung eines Rings für das in Figur 1 gezeigte Zahnimplantat

Figur 3 einen Längsschnitt durch einen Zahn, in dem das in Figur 1 gezeigte Zahnimplantat -mit Sprengring -eingesetzt ist

Figur 4 eine andere Ausführungsform des erfindungsgemäßen Zahnimplantates, bei dem das Markierungsteil im Inneren des Zahnimplantates angeordnet ist.

Figur 5 zeigt ein enossales Kieferimplantat in Form einer Knochenschraube, in welchem am Einschraubende ein Markierungsteil eingebracht ist, in starker Vergrößerung.

Figur 6 zeigt ein ankerförmiges Kieferimplantat mit eingebrachtem Markierungsteil.

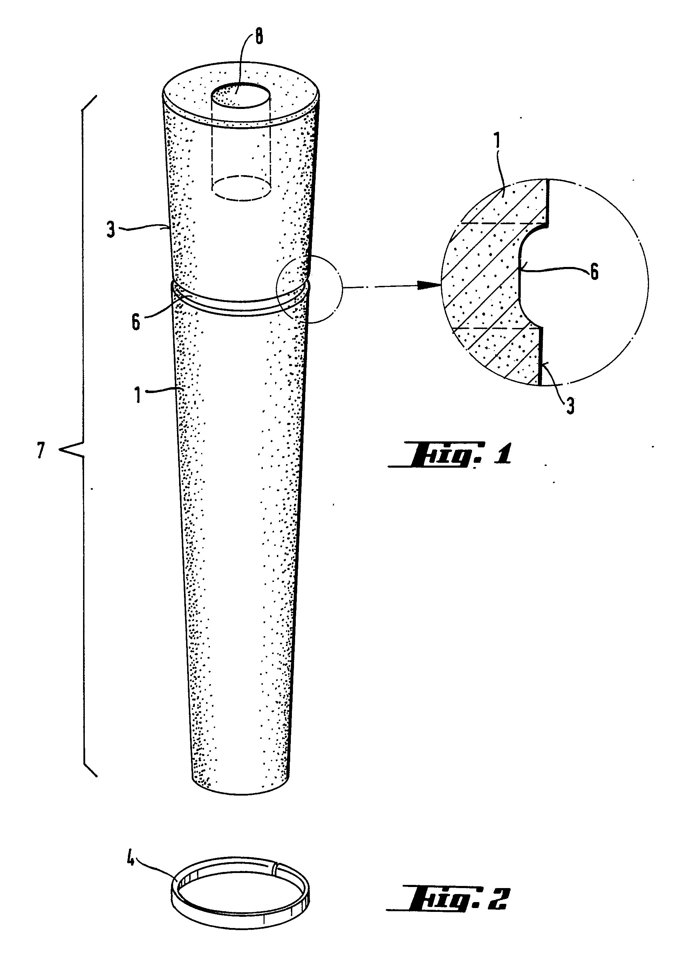

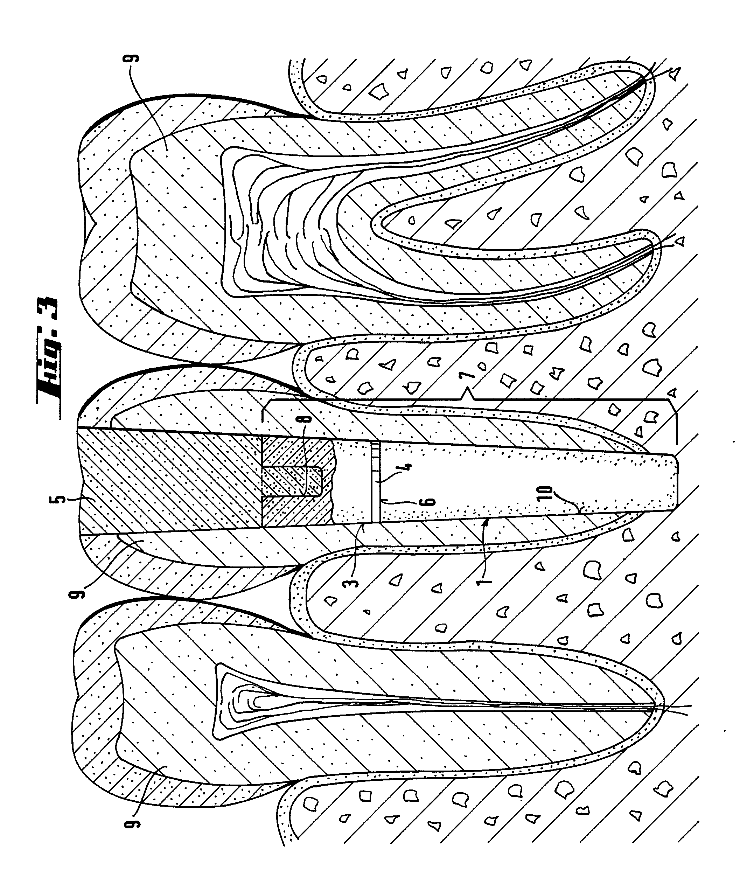

Der in Figur 1 gezeigte Körper (20) bildet zusammen mit dem in Figur 2 gezeigten Markierungsteil (4) ein erfindungsgemäßes Zahnimplantat. Auf der Oberfläche (3) ist der Körper (20) mit einer Rille (6) versehen, die zum Einsetzen des in Figur 2 gezeigten sprengringförmig ausgebildeten Markierungsteils (4) dient. Eine Öffnung (8) dient zum Angriff eines Werkzeuges beim Einsetzen des Zahnimplantates.

Figur 2 zeigt das Markierungsteil (4), das nach Art eines Sprengringes über den in Figur 1 gezeigten Körper (20) geschoben wird und in der Rille -(6) fixiert werden kann.

Figur 3 zeigt ein endodontisches Implantat (1), das aus dem in Figur 1 gezeigten Körper (20) und dem in Figur 2 gezeigten Markierungsteil (4) zusammengesetzt ist. Mit seinem Bereich (7) ist das Implantat (1) in einen Zahn (9) eingelagert. Das sprengringförmige Markierungsteil (4) sitzt in der dafür vorgesehenen Rille (6) des Körpers (20) und gestattet infolge seiner Ausbildung aus Metall die Herstellung eines kontrastreichen Röntgenbildes, nachdem das in Form eines Wurzelkanalstiftes ausgebildete Implantat (1) in den Wurzelkanal (10) des Zahnes (9) eingesetzt ist. Mit (5) ist die restliche Füllung bezeichnet, die nach Einsatz des Implantates (1) zum Verschluß des Wurzelkanalstiftes dient.

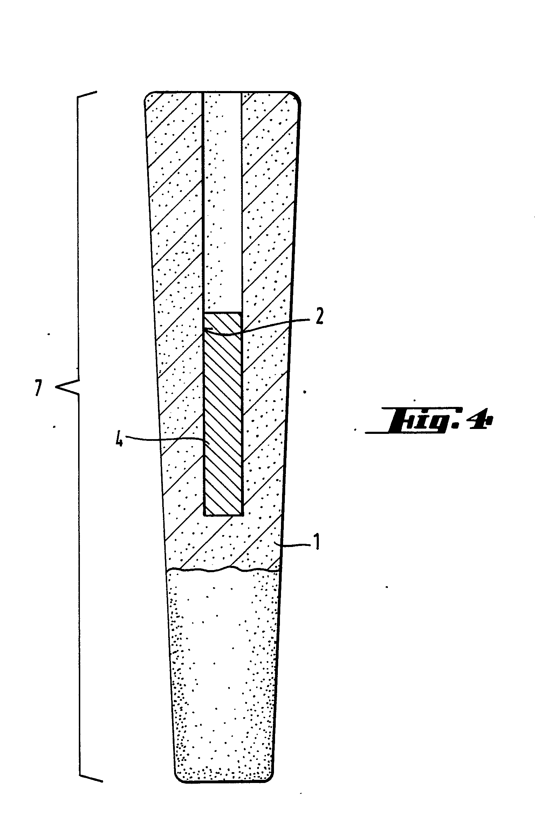

Das in Figur 4 gezeigte Implantat (1) unterscheidet sich von dem in Figur 3 gezeigten dadurch, daß das Markierungsteil (4) im Inneren -(2) des Körpers (20) angeordnet ist.

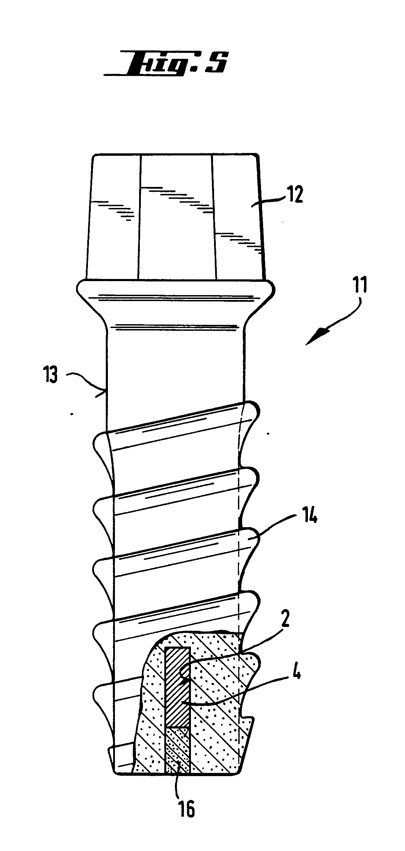

Die Figur 5 zeigt in starker Vergrößerung ein als Knochenschraube ausgebildetes Kieferimplantat (11), bestehend aus Kopfteil (12) und Körper (20) mit zylindrischem Schaftteil (13) und Gewindeteil -(14) Das Markierungsteil (4) ist hier auf der Einschraubseite in eine im Inneren (2) des Körpers -(20) angebrachte Bohrung (2) eingelagert. Diese kann im Anschluß daran mit einem kontrastarmen Kitt (16) verschlossen werden.

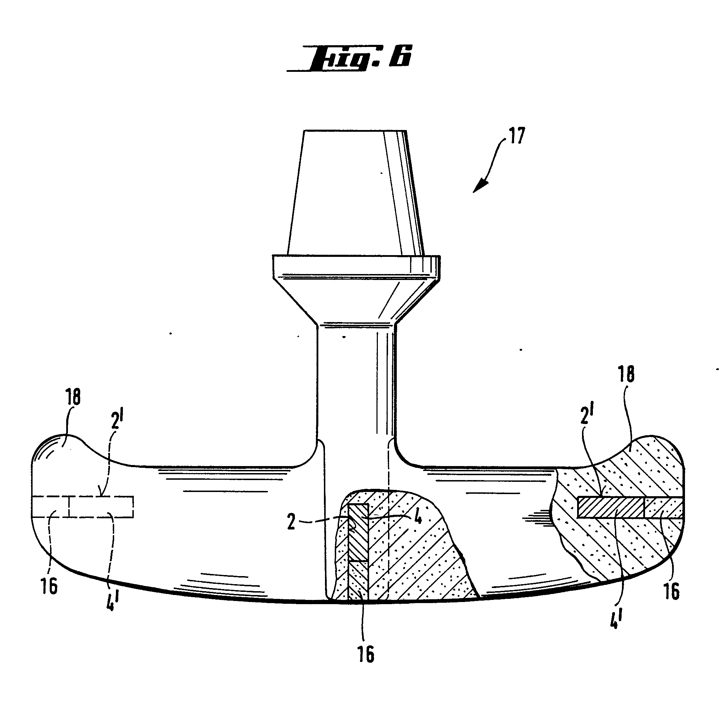

Figur 6 zeigt ein als enossales Kieferimplantat ausgebildetes Zahnimplantat (17). Der Körper (20) des Zahnimplantates (17) umfaßt den Schaft (22) und die Ankerflügel (18). Ein Markierungsteil (4) ist in einer Bohrung (21) eingebracht, bzw. sind Markierungsteile (4') alternativ in den Bohrungen (21') der Ankerflügel (18) gezeigt. Kittverschlüsse (16) dienen zum Abschluß der Bohrungen (21,21').

## Ansprüche

1. Zahnimplantat (1, 11, 17), dessen Körper -(20) aus einem biologisch verträglichen Material mit geringem Röntgenkontrast besteht, dadurch gekennzeichnet, daß der Körper (20) des Implantates (1; 11; 17) im Inneren (2) oder an einem Teil seiner Oberfläche (3) mindestens ein aus einem Material mit hohem Röntgenkontrast bestehendes Markierungsteil (4) aufweist.

2. Zahnimplantat nach Anspruch 1, dadurch gekennzeichnet, daß das biologisch verträgliche Material Keramik ist.

3. Zahnimplantat nach Anspruch 2, dadurch gekennzeichnet, daß das biologisch verträgliche Material Aluminiumoxidkeramik ist.

4. Zahnimplantat nach Anspruch 1, dadurch gekennzeichnet, daß das Material hohen Röntgenkontrastes Metall ist.

5. Zahnimplantat nach Anspruch 4, dadurch gekennzeichnet, daß das Material hohen Röntgenkontrastes Titan, Tantal, Niob, Gold, Platin oder Edelstahl ist.

6. Zahnimplantat nach einem der Ansprüche 1 bis 5, dadurch gekennzeichnet, daß das Markierungsteil (4) als Sprengring ausgebildet und in eine Rille (6) an der Oberfläche (3) eingeklemmt ist.

7. Zahnimplantat nach einem der Ansprüche 1 bis 5, dadurch gekennzeichnet, daß das Markierungsteil (4) als Beschichtung auf der Oberfläche - (3) aufgedampft ist.

8. Zahnimplantat nach einem der Ansprüche 1 bis 7, dadurch gekennzeichnet, daß das Zahnimplantat (1) als Wurzelkanalstift ausgebildet ist und das Markierungsteil (4) auf dem zur endodontischen Einlagerung vorgesehenen Bereich (7) ausgebildet ist.

9. Zahnimplantat nach einem der Ansprüche 1 bis 7, dadurch gekennzeichnet, daß das Zahnimplantat als enossales Kieferimplantat ausgebildet ist.

8

3

6

1

7

1

6

3

**Fig. 1**

4

**Fig. 2**

*Fig. 3*

0 231 838

*Fig. 4*

Fig. 5

## Fig. 6